(19) 

**Europäisches Patentamt**
**European Patent Office**
**Office européen des brevets**

(11) **EP 2 198 901 B1**

(12) **EUROPEAN PATENT SPECIFICATION**

(45) Date of publication and mention
of the grant of the patent:
**28.12.2011 Bulletin 2011/52**

(51) Int Cl.:
*A61M 1/36* (2006.01)          *A61M 1/02* (2006.01)
*B01J 20/281* (2006.01)       *G01N 30/00* (2006.01)
*G01N 30/88* (2006.01)

(21) Application number: **08830859.8**

(22) Date of filing: **08.09.2008**

(86) International application number:
**PCT/JP2008/066179**

(87) International publication number:
**WO 2009/034949 (19.03.2009 Gazette 2009/12)**

(54) **ADSORPTION COLUMN FOR PURIFYING BODY FLUID**

ADSORPTIONSSÄULE ZUR REINIGUNG VON KÖRPERFLÜSSIGKEITEN

COLONNE D'ADSORPTION POUR PURIFIER UN FLUIDE CORPOREL

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **12.09.2007 JP 2007236564**

(43) Date of publication of application:
**23.06.2010 Bulletin 2010/25**

(73) Proprietor: **Rei Medical Co., Ltd.**
**Kyoto 615-8245 (JP)**

(72) Inventors:
• **IPPOMMATSU, Masamichi**
**Kyoto-shi**
**Kyoto 615-8245 (JP)**
• **KURUSU, Chie**
**Kyoto-shi**
**Kyoto 615-8245 (JP)**
• **MIYAMOTO, Riichi**
**Kyoto-shi**
**Kyoto 615-8245 (JP)**
• **ISHII, Sayaka**
**Kyoto-shi**
**Kyoto 615-8245 (JP)**

• **NAKANISHI, Kazuki**
**Kyoto-shi**
**Kyoto 615-8245 (JP)**
• **MINAKUCHI, Hiroyoshi**
**Kyoto-shi**
**Kyoto 615-8245 (JP)**
• **YANO, Keiko**
**Kyoto-shi**
**Kyoto 615-8245 (JP)**

(74) Representative: **Treeby, Philip David William**
**R.G.C. Jenkins & Co**
**26 Caxton Street**
**London SW1H 0RJ (GB)**

(56) References cited:
DE-A1- 3 627 063          JP-A- 03 008 729
JP-A- 03 254 756          JP-A- 07 041 374
JP-A- 07 198 705          JP-A- 08 012 317
JP-A- 08 012 346          JP-A- 08 052 303
JP-A- 10 182 261          JP-A- 58 026 819
JP-A- 60 241 450          JP-A- 62 224 362
JP-A- 62 224 363          JP-A- 2004 099 418
JP-B2- 2 925 249          JP-B2- 3 100 974
JP-B2- 02 058 939         JP-B2- 03 039 736
JP-B2- 06 011 322         US-A- 4 576 927
US-A- 4 637 994           US-A1- 2004 249 082

**Description**

TECHNICAL FIELD

The present invention relates to an adsorption column for body fluid purification provided as a porous support having a functional group which binds specifically to a substance to be adsorbed immobilized on the surface thereof. Particularly, the present invention relates to an adsorption column for an apheresis treatment, which aims to remove a predisposing factor such as LDL (low-density lipoprotein) in the blood by adsorption.

BACKGROUND ART

An apheresis treatment is a treatment method intended to ameliorate a pathological condition by removing humoral factors (such as a protein, an antibody present in the blood as a conjugate with a protein, and an immune-related substance including a cytokine) and cells (such as a lymphocyte, a granulocyte, and a virus) which could cause a disease from the blood by extracorporeal circulation.

Among lipoproteins present in the blood, LDL contains cholesterol in abundance, and it is well known that hyper-low-density-lipoproteinemia, which is manifested as an elevation in the blood concentration of LDL, increases a risk of arteriosclerosis and increases risks of myocardial infarction and cerebral infarction. An LDL apheresis treatment is practiced as one of the treatment methods for hyperlow-density-lipoproteinemia; however, a current situation is that it is applied exclusively to severely affected patients who are no longer responsive to drug therapy among patients with familial (hereditary) hyper-low-density-lipoproteinemia, obstructive arteriosclerosis, focal glomerulosclerosis, and the like, due to high medical costs and great burden imposed on the patients.

The LDL apheresis treatment is an apheresis treatment which employs an adsorption method. The LDL apheresis treatment is a treatment method in which the blood or the plasma of a patient is perfused through an adsorption column provided as a support having a functional group having an affinity to LDL, which is a substance to be adsorbed, immobilized on the surface thereof, to remove LDL from the extracorporeally-circulated blood of the patient by adsorption. As an LDL adsorption column, Liposorber (registered trademark), a product of Kaneka Corporation (refer to Non-Patent Document 1 described below), DALI system, a product of Fresenius Medical Care (Germany) (refer to Patent Document 1 described below), and the like are available. The LDL adsorption columns described above are constituted by modifying the surface of a support composed of a beaded porous cellulose gel or beaded polyacrylamide having numerous pores with a chained polyhydric acid such as dextran sulfate or a polyacrylic acid as a functional group (ligand) having an affinity to LDL and immobilizing the above materials thereon, and filling the surface-modified bead thus obtained in a column container. The diameter of the bead is approximately 250 $\mu$m in a case of the direct hemoperfusion, and approximately 50 $\mu$m in a case of the plasma perfusion.

Non-Patent Document 1: Nobutaka TANI, "Development of Blood Adsorption Device", the Japanese Journal of Medical Instrumentation, Vol. 58, No. 6, pp. 266-273, 1988
Patent Document 1: Japanese Examined Patent Application Publication No. 3-254756
Patent Document 2: Japanese Examined Patent Application Publication No. 3-39736
Patent Document 3: Japanese Patent Application Laid-Open Publication No. 7-41374

US 4,637,994 discloses an adsorbent for removing low and/or very low density lipoprotein from body fluid in extracorporeal circulation treatment, which comprises a water-insoluble porous hard gel with exclusion limit of $10^6$ to $10^9$ daltons on which a sulfated compound is immobilized by a covalent linkage.

DISCLOSURE OF THE INVENTION

PROBLEMS TO BE SOLVED BY THE INVENTION

However, an LDL adsorption column practically used at present in which a porous cellulose gel bead or the like is employed as a support has such problems as shown below.
Firstly, a porous cellulose gel bead is in the form of a thread ball of tangled cellulose fibers and a distribution of the diameter of a pore in the bead is 0.1 to 1 $\mu$m, which is as large and broad as 4 to 40 times the diameter of a spherical LDL molecule (approximately 26 to 27 nm) (refer to Non-Patent Document 1 (4.1 Support)). For this reason, the surface area per pore volume becomes small as the diameter of a pore is enlarged, leading to a decrease in the adsorption efficiency. Also, controlling the distribution of the diameter of a pore is difficult because the bead is in the form of a thread ball, and in a case when the diameter of a pore is smaller than approximately the diameter of an LDL molecule, LDL will not be adsorbed to the pore surface, leading to a decrease in the adsorption efficiency.

Secondary, while the blood or the plasma flowed into an adsorption column will flow through spaces between beads as channels by a blood stream, transport of LDL, which is a substance to be adsorbed, to the pore surface inside the bead depends on diffusion, and further, a support is in the form of a bead and the diameter thereof is larger with respect to the diameter of the pore. For this reason, LDL is only adsorbed to the pore surface near the surface of the bead, and hence, the total pore surface area covering from near the surface of the bead to the deep part thereof cannot be fully effectively utilized.

Thirdly, when the bead diameter is made small with an aim to improve the second problem posed as above, spaces between beaded supports are constricted, and channel resistance of a blood stream is raised and a pressure loss is increased. Hence, a flow volume of the blood stream cannot be increased. If it is supposed that there is a case in which a column container is densely packed with a beaded support, the diameter of a channel running through a constricted site in spaces between beaded supports will be narrowed to approximately 15% of the bead diameter, and because the constricted site is surrounded from four directions by the beaded support, a channel coming into the constricted site from one direction will be greatly bent by an opposite beaded support. An increase in pressure loss is considered to be caused by such reasons as stated above.

Fourthly, in a case of a soft gel such as a porous cellulose gel, a pressure loss is further increased, and inflow of a large volume of the blood or the plasma causes consolidation, due to which the blood or the plasma stops flowing at a certain flow volume or more.

Fifthly, because a polymer support such as a porous cellulose gel contains a trace amount of additives and the like, adsorption of proteins in the blood and activation of a blood coagulation mechanism are promoted.

Summing up the above, an LDL adsorption column practically used at present in which a porous cellulose gel bead is employed as a support is accompanied by two types of problems; namely, a problem which is attributable to that a support is a porous cellulose gel (first, fourth, and fifth problems), and a problem which is attributable to that a support is in the form of a bead (first, second, and third problems).

A solution method in which a support is constituted by an inorganic porous body is proposed by the above-described Patent Document 2 with respect to the fourth problem which is attributable to that a support is a porous cellulose gel bead, namely, such a problem that a pressure loss is increased and the blood or the plasma stops flowing at a certain flow volume or more. However, in Patent Document 2, as a support, a porous glass bead (having a particle size of 80 to 120 mesh) and an agarose gel bead (having a particle size of 50 to 100 mesh) are compared in regard to a correlation between pressure and flow velocity, and it is merely elucidated that no consolidation is caused and the flow velocity increases as the pressure is raised in a case of the porous glass bead, whereas the flow velocity does not increase in a case of the agarose gel bead (refer to Fig. 1 in Patent Document 2). At this point, the bead diameter of the porous glass bead is defined as large as approximately 250 $\mu$m on the assumption of direct hemoperfusion, and the diameter of a channel running through a constricted site in spaces between beaded supports is also large. Accordingly, absolutely neither consideration nor evaluation is provided on an effect exerted by the bead diameter on the pressure loss and the flow velocity. Furthermore, while it is described that "an arbitrary form such as a granular form, a fibrous form, a membranous form, or a hollow fiber form can be selected as a form of a support" in Patent Document 2, an actual evaluation was conducted with a beaded (spherical) support, and absolutely neither consideration nor evaluation is provided on an effect exerted by the form of a support on the pressure loss and the flow velocity.

Further, a performance evaluation was carried out with regard to an LDL adsorbent employing a beaded support in Patent Document 2; however, it is a static adsorption test in which the plasma is added to a test cylinder containing a spherical adsorbent and stirred, and the mixture is left to stand at 20°C for 15 minutes, after which a concentration of LDL in a supernatant is measured, which is not a dynamic adsorption test performed in a condition in which the blood or the plasma is flowed through, which conforms to a usage pattern of an LDL adsorption column practically used at present. Hence, an effect of the bead diameter of a beaded support, namely, an effect exerted on the pressure loss in a condition in which the blood or the plasma is flowed through the adsorption column is not sufficiently evaluated. Further, because absolutely no evaluation is conducted by a dynamic adsorption test, absolutely neither consideration nor evaluation is provided on an effect exerted by the bead diameter and the form of a support on adsorbability when an LDL adsorption column is in practical use.

Furthermore, due to the first and the second problems as described above, a saturated adsorption amount of LDL is confined to approximately 6 mg/mL in an LDL adsorption column available at present. Thus, with respect to a column capacity, a column with a size of 400 to 1000 mL is necessary in a case of a non-recyclable column, and in a case of a recyclable column, a complex recycling system using two columns with a size of 150 mL each is necessary. Moreover, because an elapsed time to reach saturated adsorption is long, a time required for treatment is also as long as two to three hours, and hence, a great burden is imposed on a patient.

The present invention is completed in view of the above-described problems, and an object of the present invention is to provide an adsorption column for body fluid purification by which the saturated adsorption amount of a substance to be adsorbed which includes at least a low density lipoprotein can be increased or the time required for reaching the saturated adsorption can be shortened, and therefore, the column capacity can be considerably reduced and the treatment time can be considerably shortened. Also, another object of the present invention is to provide an adsorption column for body fluid purification by which the column capacity can be reduced to approximately one column with a size of 200 mL and the time required for treatment can be reduced to approximately 30 minutes, in a case when a substance to be adsorbed is LDL.

MEANS FOR SOLVING THE PROBLEMS

The present inventors conducted a thorough study focusing on that a material and a form of a support of an LDL adsorption column available at present have room for improvement. As a result, they found that problems accompanying a conventional adsorption column can be solved and a considerable increase in performance can be realized with an adsorption column for body fluid purification provided as a porous support having a functional group which binds specifically to a substance to be adsorbed immobilized on the surface thereof, by substituting a constitution in which a beaded adsorbent provided as a beaded porous support having a functional group immobilized thereto is filled in a column as conventionally used, by employing, as a porous support, an integrated porous support which contains an inorganic skeleton composed of a silica gel or silica glass having a three-dimensional network structure, and through-pores having a three-dimensional network form, which are formed in the voids of the skeleton, and which contains micropores with a diameter smaller than that of the through-pore which are dispersedly formed in the skeleton, and by optimizing average diameters of the through-pores and the micropores.

That is, a first characteristic of the adsorption column according to the present invention provided to achieve the above-described objects is that it is an adsorption column for body fluid purification comprising a porous support having a functional group immobilized on a surface of the porous support, the functional group specifically binding to a substance to be adsorbed including at least a low density lipoprotein, wherein the porous support includes: a skeleton made of a silica gel or silica glass having a three-dimensional network structure; and through-pores of an average diameter in a range of 1 $\mu$m or more to less than 4 $\mu$m as measured by a mercury intrusion method, the through-pores being formed in the voids of the skeleton and having a three-dimensional network form; and micropores of an average diameter of larger than a maximum length of the substance to be adsorbed in the body fluid purification as measured by a mercury intrusion method, the micropores penetrating from a surface of the skeleton to an inner part thereof and being dispersedly formed. At this point, the surface of the porous support corresponds to each inner wall surface of the through-pores and the micropores of the porous support. Also, the surface of the skeleton is equivalent to an inner wall surface of the through-pore. Wherein, the average diameter of the micropore as measured by a mercury intrusion method is 27 nm or more and 200 nm or less.

Further, a second, preferred characteristic of the adsorption column for body fluid purification of the present invention is that it is the adsorption column for body fluid purification having the above-described first characteristic, wherein the average diameter of the micropores as measured by a mercury intrusion method is twice or more as large as the maximum length of the substance to be adsorbed.

Further, a third, preferred characteristic of the adsorption column for body fluid purification of the present invention is that it is the adsorption column for body fluid purification having any one of the above-described characteristics, wherein the functional group is dextran sulfate or a salt thereof, a polyacrylic acid or a salt thereof, or another chained polyhydric acid or a salt thereof having an affinity to specifically bind to a low density lipoprotein.

Further, a fourth, preferred characteristic of the adsorption column for body fluid purification of the present invention is that it is the adsorption column for body fluid purification having any one of the above-described characteristics, wherein the porous support is synthesized by a spinodal decomposition sol-gel method.

Further, a fifth, preferred characteristic of the adsorption column for body fluid purification of the present invention is that it is the adsorption column for body fluid purification having any one of the above-described first characteristic to fifth characteristic, wherein the porous support is formed into a columnar shape and contained in a cylindrical container in such a way that a columnar lateral surface of the porous support is closely contacted with an inner wall surface of the cylindrical container, the cylindrical container having an opening at least in a part of each of both end surfaces, and the opening in one end surface of the cylindrical container is communicated with the opening in the other end surface thereof via the through-pore of the porous support.

Further, a sixth, preferred characteristic of the adsorption column for body fluid purification of the present invention is that it is the adsorption column for body fluid purification having any one of the above-described first characteristic to fifth characteristic, wherein the porous support is formed into a cylindrical shape and contained in a cylindrical container in such a way that at least a part of a cylindrical outer lateral surface of the porous support is spaced from an inner wall surface of the cylindrical container, the cylindrical container having an opening at least in a part of each of both end surfaces, the opening in one end surface of the cylindrical container is communicated with both of an outer lateral part and an inner lateral part of the porous support, and the opening in the other end surface of the cylindrical container is communicated with either one of the outer lateral part and the inner lateral part of the porous support.

According to the adsorption column for body fluid purification of the above-described first characteristic, a problem which is attributable to that a porous support is a polymer or a soft gel and a problem which is attributable to that a porous support is in the form of a bead, as observed in a conventional adsorption column, are solved simultaneously since the adsorption column for body fluid purification employs an inorganic silica gel or silica glass as a porous support.

Specifically, by employing an integrated porous support which contains a skeleton having a three-dimensional network structure and through-pores having a three-dimensional network form, which are formed outside the skeleton, the through-pores, which serve as channels for a body fluid to be subjected for purification of a body fluid (for example, the plasma), are communicated in a three-dimensional network manner. Therefore, even when the diameter of the through-pores is the same as the diameter of channels running through a constricted site in spaces between beaded supports in a case when the beaded support is densely packed in the column container, channel resistance can be suppressed low and a pressure loss can be suppressed low in comparison with the beaded support. Furthermore, given that transport of a substance to be adsorbed within a micropore relies on diffusion both in the beaded support and the skeleton having a three-dimensional network structure, if it is supposed that exclusively a functional group immobilized in micropores near the surface of each support functions effectively, regarding a three-dimensional form of the support, a surface area of the micropore in which a functional group effectively functions can be more increased with a three-dimensional network structure having a larger surface area per unit volume than with the form of a bead (sphere) having a small surface area. Accordingly, in the same channel diameter, a pressure loss is suppressed low while an effective micropore area is enlarged with a three-dimensional network structure than with a beaded support. In other words, the channel diameter can be made smaller while the effective micropore area can be expanded more with a three-dimensional network structure than with a beaded support.

It is to be noted that the below-described results of evaluation of a dynamic adsorbability targeting LDL as a substance to be adsorbed revealed that when an average diameter of the through-pore was less than 1 $\mu$m, a pressure loss became too high and a body fluid flow was interfered, leading to a reduction in an efficiency of body fluid purification, namely an adsorbability of LDL, while, in contrast, when an average diameter of the through-pore was 4 $\mu$m or more, an effective area of the micropore decreased, leading to a reduction in an efficiency of body fluid purification, namely an adsorbability of LDL. Accordingly, if the average diameter of the through-pore is within a range of 1 $\mu$m or more to less than 4 $\mu$m, it can be attempted to provide a considerably improved dynamic adsorbability of approximately 5 or more times the adsorbability of a conventional adsorption column, at least with respect to LDL, achieved in a condition in which a body fluid to be treated is flowed through an adsorption column.

Furthermore, by setting an average diameter of the micropore dispersedly formed in a skeleton to be larger than a maximum length of a substance to be adsorbed, a functional group immobilized on two kinds of the surfaces including a surface exposed toward a through-pore in a skeleton (surface of the through-pore) and a surface exposed toward a micropore in a skeleton (surface of the micropore) can be made to adsorb a substance to be adsorbed. That is, if a diameter of the micropore is smaller than a maximum length of a substance to be adsorbed (which is a diameter or a greatest dimension in a case when the substance to be adsorbed is spherical, oval sphere-shaped, or disk-shaped, which corresponds to a case in which the substance to be adsorbed is LDL), transport of the substance to be adsorbed to the inside of the micropore is either disabled or made extremely difficult. Hence, adsorption of the substance to be adsorbed exclusively relies on a functional group immobilized on the surface of the through-pore. In contrast, if the diameter of the micropore is larger than a maximum length of a substance to be adsorbed, at least one substance to be adsorbed is transported to the inside of the micropore and enabled to adsorb to a functional group immobilized on the surface of the micropore, thereby an effective surface area of a skeleton available for adsorption can be increased. However, a micropore having a diameter approximately as large as the diameter of the through-pore is no longer a micropore but regarded as equivalent to the through-pore, and such a micropore no longer falls into a condition such that micropores are distributed on the surface of the skeleton. Accordingly, a characteristic of a porous support having a three-dimensional network structure used in the present invention lies in a point that it is made into a hierarchical porous structure by two kinds of diameters including a through-pore having a three-dimensional network form and a micropore having a smaller diameter than that of the through-pore. In other words, utilization of the above-described characteristic of the porous structure enables setting of a condition by which a considerable improvement in performance can be attempted with respect to an adsorption column employing a conventional beaded porous body.

Furthermore, in a case in which a channel diameter and a diameter of the through-pore are the same when a beaded support is densely packed in a column container, because the diameter can be made smaller with a skeleton having a three-dimensional network structure than with a beaded support, an extension length, which is a length of the micropore extending from the surface of a support toward an inner part thereof, is shorter in the skeleton having a three-dimensional network structure than in the beaded support. Thus, a transport distance of a substance to be adsorbed to the inside of the micropore by diffusion is shortened, leading to an improvement in an efficiency of adsorption to a functional group in the micropore. In other words, an elapsed time to reach saturated adsorption is shortened in comparison with a conventional adsorption column employing a beaded support, and a high-performance adsorption column can be fully exploited in a short time, by which a time required for treatment is shortened and a burden imposed on a patient can be considerably reduced.

Further, according to the adsorption column for body fluid purification of the second characteristic, by setting an average diameter of the micropore dispersedly formed on the surface of a skeleton to be twice or more as large as a maximum length of a substance to be adsorbed, it is made possible to serve a functional group immobilized on the surface of the micropore more efficiently to adsorption of a substance to be adsorbed. In other words, if a diameter of the micropore is twice or more as large as a maximum length of a substance to be adsorbed, a substance to be adsorbed which has been adsorbed to a functional group immobilized on the surface of the micropore is less likely to interfere transport of another substance to be adsorbed into the same micropore and adsorption of the same to another functional group. Thus, as the diameter of the micropore becomes large, the depth of the micropore (distance from the surface of a skeleton) to be effectively utilized for adsorption is increased, and a substance to be adsorbed which is transported to the inside of the micropore and adsorbed is increased. However, if an average diameter of the micropore is large, the number of the micropores per unit surface area of a skeleton is decreased; hence, even if the diameter of the micropore is made larger than a certain range, adsorbability is not increased. It is to be noted that an upper limit of the certain range is not flatly set because it depends on the diameter of the through-pore and a size of a substance to be adsorbed; however, it is considered to be approximately 6 to 10 times as large as a maximum length of a substance to be adsorbed based on the results of measurement obtained by examples below. However, the diameter of the micropore can be made larger than the certain range as long as adsorbability is not considerably reduced.

Further, according to the above-described adsorption column for body fluid purification of the third and the fourth characteristics, a favorable adsorbability can be obtained when a substance to be adsorbed is LDL (having the diameter of approximately 26 to 27 nm).

Further, according to the above-described adsorption column for body fluid purification of the fifth characteristic, utilization of a spinodal decomposition sol-gel method improves controllability of the diameters of the through-pore and the micropore. Thus, a high-performance adsorption column in which the diameters of the through-pore and the micropore are adjusted within an appropriate range can be provided.

Further, according to the above-described adsorption column for body fluid purification of the sixth characteristic, it is possible to provide an adsorption column suitable for a case in which the plasma obtained after separating blood cell components from the blood is used as a body fluid containing a substance to be adsorbed.

Further, according to the above-described adsorption column for body fluid purification of the seventh characteristic, it is possible to provide an adsorption column suitable for a case in which the blood is used as a body fluid containing a substance to be adsorbed. In that case, because an average diameter of the through-pore is within a range of 1 μm or more to less than 4 μm, a blood cell which is larger than the diameter of the through-pore such as an erythrocyte (the diameter of a human erythrocyte is approximately 8 μm) or a leukocyte (the diameter of a human leukocyte is approximately 7 to 25 μm) in the blood supplied from an opening in the other end surface of a cylindrical container to either one of an outer lateral part and an inner lateral part of a porous support (entry side) is directly transported to an opening in one end surface of the cylindrical container without passing through the porous support. Whereas, while a plasma component lacking a blood cell which is larger than the diameter of the through-pore (but containing a part of platelets having a small diameter) is passed through the porous support from one side (entry side) to the other side (exit side), a substance to be adsorbed present in the plasma is adsorbed to a functional group immobilized to the porous support, and the plasma component is transported from the exit side of the porous support to an opening in one end surface of the cylindrical container to be joined with the blood containing a blood cell, and then can be discharged. As such a constitution as stated above is provided by the above-described adsorption column for body fluid purification of the seventh characteristic, body fluid purification by which a substance to be adsorbed is removed by adsorption is made possible without separating blood cell components from the blood.

BRIEF DESCRIPTION OF THE DRAWINGS

Fig. 1 is a constitutional diagram schematically showing an overview of a constitution of a first embodiment of the adsorption column for body fluid purification according to the present invention.
Fig. 2 is a cross-sectional view of a principal part schematically showing a structure of an adsorbent of the adsorption column for body fluid purification according to the present invention.
Fig. 3 is a cross-sectional view of a principal part schematically showing a structure of a porous support constituting an adsorbent of the adsorption column for body fluid purification according to the present invention.
Fig. 4 is a SEM photograph of a porous support constituting an adsorbent of the adsorption column for body fluid purification according to the present invention.
Fig. 5 is a list summarizing the results of comparative evaluation of adsorbability between a test column filled with an adsorbent of the adsorption column for body fluid purification according to the present invention and a test column filled with a beaded adsorbent of Liposorber (type: LA-15).

Fig. 6 is a graph showing the results of comparative evaluation of adsorbability to the total cholesterol between a test column filled with an adsorbent of the adsorption column for body fluid purification according to the present invention and a test column filled with a beaded adsorbent of Liposorber (type: LA-15).

Fig. 7 is a graph showing the results of comparative evaluation of a temporal change of the adsorption amount of the total cholesterol between a test column filled with an adsorbent of the adsorption column for body fluid purification according to the present invention and a test column filled with a beaded adsorbent of Liposorber (type: LA-15).

Fig. 8 is a table showing the adsorption selectivity of a test column filled with an adsorbent of the adsorption column for body fluid purification according to the present invention.

Fig. 9 is a graph showing the adsorption selectivity of a test column filled with an adsorbent of the adsorption column for body fluid purification according to the present invention.

Fig. 10 is a graph showing a dependency of adsorbability of a test column filled with an adsorbent of the adsorption column for body fluid purification according to the present invention with respect to LDL on an average diameter of the through-pore.

Fig. 11 is a graph showing a dependency of adsorbability of a test column filled with an adsorbent of the adsorption column for body fluid purification according to the present invention with respect to LDL on an average diameter of the micropore.

Fig. 12 is a constitutional diagram schematically showing an overview of a constitution of a second embodiment of the adsorption column for body fluid purification according to the present invention.

BEST MODES FOR CARRYING OUT THE INVENTION

Embodiments of the adsorption column for body fluid purification according to the present invention (hereinafter appropriately called "the apparatus of the present invention") are described based on the drawings.

First embodiment

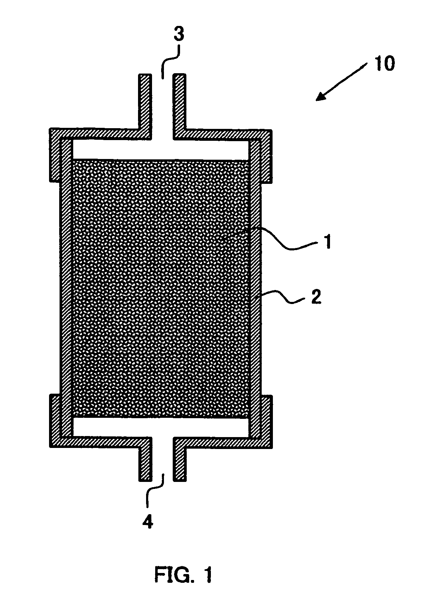

As shown in Fig. 1, an apparatus of the present invention 10 is constituted in such a way that a columnar adsorbent 1 is contained in a cylindrical container 2. Openings 3 and 4 are each formed in each of the end surfaces of the cylindrical container 2, and the opening 3 serves as an inlet for a body fluid to be treated (postulated to be the plasma in the present embodiment), while the other opening 4 serves as an outlet for a treated body fluid.

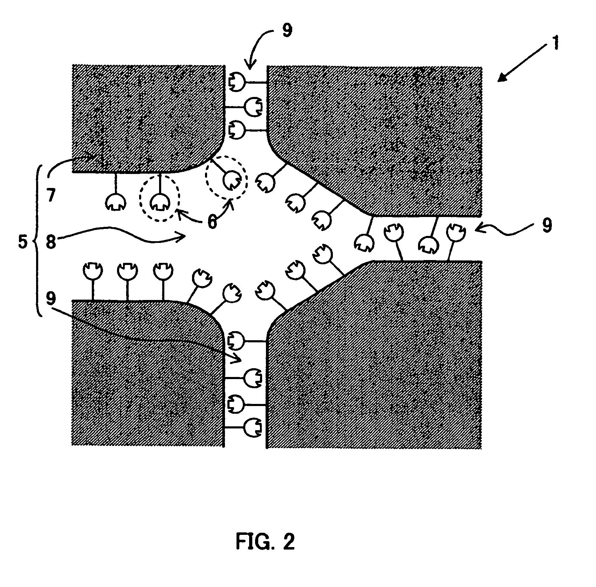

The adsorbent 1, which is a major component part of the apparatus of the present invention 10, is, as schematically shown in Fig. 2, provided by modifying the surface of an inorganic porous support 5, which is formed in a columnar shape, with a functional group 6 which specifically binds to a substance to be adsorbed and immobilizing the functional group 6 thereon. In the present embodiment, LDL in the blood is postulated to be the substance to be adsorbed, and as the functional group 6, dextran sulfate or a salt thereof, a polyacrylic acid or a salt thereof, or another chained polyhydric acid or a salt thereof having an affinity to LDL is used.

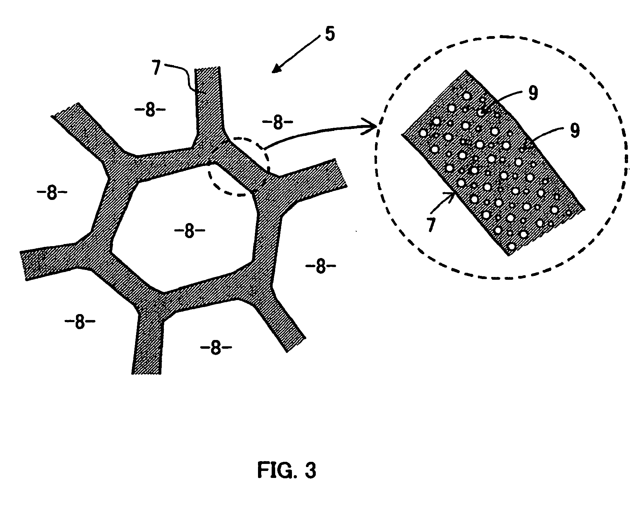

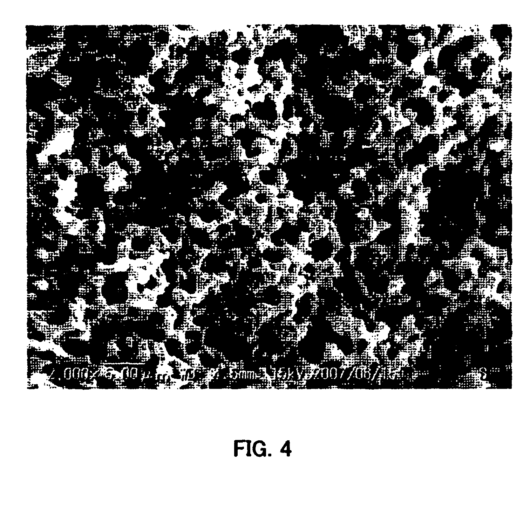

The inorganic porous support 5, which constitutes the adsorbent 1, is, as schematically shown in Fig. 3, provided by having a skeleton 7, which has an integrated structure in a three-dimensional network manner, and through-pores 8 with an average pore diameter in a range of 1 $\mu$m or more to less than 4 $\mu$m, which is provided in a three-dimensional network manner and formed in the voids of the skeleton 7. Further, micropores 9 with an average diameter of 27 nm or more and 200 nm or less, which is equal to or larger than the diameter (corresponding to a maximum length) of LDL as a substance to be adsorbed, which is 26 to 27 nm, are dispersedly formed on the surface of the skeleton 7. Accordingly, the porous support 5 used in the apparatus of the present invention 10 is provided as a double pore structure composed of two kinds of pores having different average diameters (the through-pore 8 and the micropore 9). Fig. 4 shows an example of a SEM (scanning electron microscope) photograph of the porous support 5 used in the apparatus of the present invention 10.

A method for producing the adsorbent 1 provided by the porous support 5 having dextran sulfate as the functional group 6 immobilized on the surface thereof is then described. Firstly, a method for synthesizing the porous support 5 is described. It is to be noted that the synthetic method employed in this embodiment uses a spinodal decomposition sol-gel method based on the principle disclosed in "Method for Synthesizing Inorganic Porous Body" in the above-described Patent Document 3.

Firstly, polyethylene glycol (molecular weight: 100000) is dissolved at a range of 0.9 g to 1.1 g with respect to 9 mL of 1 M (volume molar concentration) aqueous solution of nitric acid, to which 7 mL of tetraethoxysilane is added. The mixture is stirred to homogeneity, then left to stand at 40°C in a thermostat bath overnight to allow gelation to proceed (step 1). Subsequently, the gel thus obtained is immersed in 1 M aqueous ammonia, and a reaction is allowed to proceed at 90°C for three days (step 2). Subsequently, the gel is dried and heated to obtain a porous support composed of a silica gel or silica glass (step 3). It is to be noted that, when a porous support is produced under the above-described conditions, an average diameter of the through-pore contained in the porous support is approximately 1 μm to 5 μm as measured by a mercury intrusion method. Also, an average diameter of the micropore is approximately 45 nm as measured by a mercury intrusion method.

More specifically, when the porous support 5 is produced under the same conditions as above except using the varied additive amounts of polyethylene glycol of 1.1 g, 1.0 g, and 0.9 g in the above step 1, an average diameter of the through-pore contained in each porous support is obtained as 1 μm, 3 μm, and 5 μm, respectively. From the above, an average diameter of the through-pore can be adjusted by adjusting an additive amount of polyethylene glycol to be added to an aqueous solution of nitric acid.

Further, an average diameter of the micropore (as measured by a mercury intrusion method) varies within a range of approximately 10 nm to 200 nm by adjusting a treatment temperature in the above step 2 within a range of approximately 40°C to 250°C. Accordingly, an average diameter of the micropore can be set to be within an optimal range by adjusting a treatment temperature in the above step 2.

The above-described method employs a sol-gel method, in which solutions of organic and inorganic metal compounds are mixed and gelation is allowed to proceed by a hydrolysis reaction and a dehydration condensation reaction of an alkoxide, after which the gel thus obtained is dried and heated to produce an oxide solid. Furthermore, the above-described method utilizes a characteristic such that when an organic polymer is added to a starting solution, a split-phased structure which is produced by spinodal decomposition of a silica polymer produced along with progress of gelation and a solvent containing the organic polymer is immobilized by gelation, thereby a porous gel having micropores of μm-order is produced. That is, according to the method described above, the porous support can be produced by causing spinodal decomposition while employing a sol-gel method (a spinodal decomposition sol-gel method).

It is to be noted that a total porosity (as measured by a mercury intrusion method) of the through-pores and the micropores in the porous support produced through the above-described steps 1 to 3 is within a range of approximately 50% to 70%, which is approximately 1.5 times as large as the porosity (approximately 40%) of Liposorber (type: LA-15), a product of Kaneka Corporation, which serves as a conventional adsorption column for LDL.

A method for modifying the surface of the porous support produced through the above-described steps 1 to 3 with dextran sulfate sodium having an affinity to LDL as a functional group and immobilizing the dextran sulfate sodium thereon is then described.

Firstly, the porous support produced through the above-described steps 1 to 3 is refluxed in a 10% toluene solution of γ-aminopropyltriethoxysilane for three hours, and then washed with ethanol to obtain γ-aminopropylated porous support (step 4). The immobilized amount of a γ-aminopropyl group is 0.3 mmol/mL under the above-described conditions as measured by an organic trace element analysis.

Then, 10 mL of a 1/300 M phosphate buffered aqueous solution (pH 7.0) in which 500 mg of dextran sulfate sodium is dissolved is produced with respect to 5 mL of the γ-aminopropyl porous support obtained through the above-described step 4. The γ-aminopropyl porous support is immersed into the aqueous solution and shaken at 60°C for three days (step 5). After the reaction, the porous support thus obtained is immersed in a 1% aqueous solution of NaBH$_4$ for 15 minutes, followed by sequential washing with each of pure water and physiological saline (in the order as described) to obtain an adsorbent provided as a porous support having dextran sulfate sodium immobilized on the surface thereof (step 6). The immobilized amount of dextran sulfate sodium is 10 mg/mL under the above-described conditions as measured by fluorescent X-ray diffractometry.

Then, a dynamic test method for comparative evaluation of adsorbability to LDL between the adsorbent obtained through the above-described steps 1 to 6 and a beaded adsorbent of Liposorber (type: LA-15), a product of Kaneka Corporation, which serves as a conventional adsorption column for LDL, is described. In the following description, the adsorbent obtained through the above-described steps 1 to 6 is appropriately called "the adsorbent of the present invention" to distinguish it from a conventional beaded adsorbent. It is to be noted that a functional group in the beaded adsorbent of Liposorber (type: LA-15) is dextran sulfate, which is the same as the functional group of the adsorbent of the apparatus of the present invention.

Firstly, a plurality of samples of the adsorbent with different average diameters of each of the through-pore and the micropore, and the beaded adsorbent of Liposorber (type: LA-15) are filled, 1 mL each, in small glass column containers. The column containers thus prepared are served as test columns. Then, pure water and physiological saline, 10 mL each, are sequentially flowed through each column at a flow rate of 0.5 mL/minute to wash each test column. Subsequently, 5 mL of fresh human plasma is flowed through at a predetermined flow velocity, and an average flow velocity (mL/minute), an elution time (minute), an amount of eluted liquid (mL), a total plasma cholesterol concentration before and after the flowing through (mg/dL), a plasma HDL (high density lipoprotein) cholesterol concentration before and after the flowing through (mg/dL), a plasma triglyceride concentration before and after the flowing through (mg/dL), and a plasma LDL concentration before and after the flowing through (mg/dL) are measured for each test column. Thereafter, an adsorption capacity A (mg/mL) for the total cholesterol, HDL, triglyceride, and LDL cholesterol per mL of the adsorbent were calculated for each test column based on a calculation formula shown in Mathematical formula 1 below. In Mathematical formula 1, Ci and Co represent the concentration (mg/dL) before and after the flowing through, and V represents the amount of eluted liquid (mL), respectively.
$$A = (5 \times Ci \cdot V \times Co)/100 \qquad\qquad (1)$$

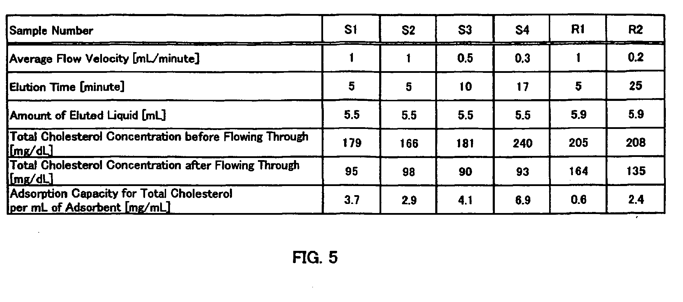

The average flow velocity (mL/minute), the elution time (minute), the amount of eluted liquid (mL), the total plasma cholesterol concentration before and after the flowing through (mg/dL), and the adsorption capacity A (mg/mL) for the total cholesterol per mL of the adsorbent of each test column (sample numbers S1 to S4, R1, and R2) are summarized in the list in Fig. 5. It is to be noted that the sample numbers S1 to S4 refer to test columns filled with the adsorbent (hereinafter appropriately called "the sample "), and average diameters of each of the through-pore and the micropore of each test column are also shown together. The sample numbers R1 and R2 refer to test columns filled with the beaded adsorbent of Liposorber (type: LA-15) (hereinafter appropriately called "the comparative sample"). It is to be noted that average diameters of each of the through-pore and the micropore of each test column of the samples S1 to S4 are within an appropriate range as described below.

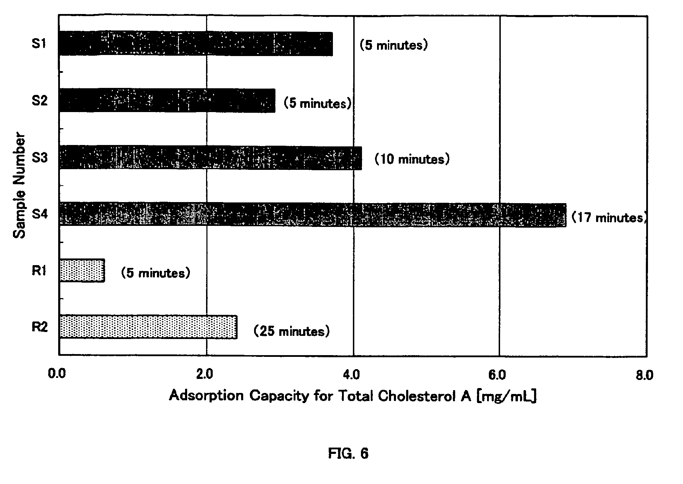

Fig. 6 shows a graph of an adsorption capacity A (mg/mL) of the total cholesterol of each test column (the sample numbers S1 to S4, R1, and R2). It is to be noted that the parenthesized values in the graph show the elution time (minute), which represents a treatment time required for adsorption.

As clearly shown in Fig. 6, in the case of the samples , the adsorbability to the total cholesterol is not reduced and is superior in comparison with the comparative samples despite that the elution time (treatment time) is shortened by increasing the flow velocity. Particularly, comparing the samples S1 and S2 with the comparative sample R1, it is found that the adsorption amount of the total cholesterol of S1 and S2 is approximately 5 times as large as that of R1. As a result, it is found that a column content can be reduced to approximately one 200 mL-column by the apparatus of the present invention, in the case when a substance to be adsorbed is LDL.

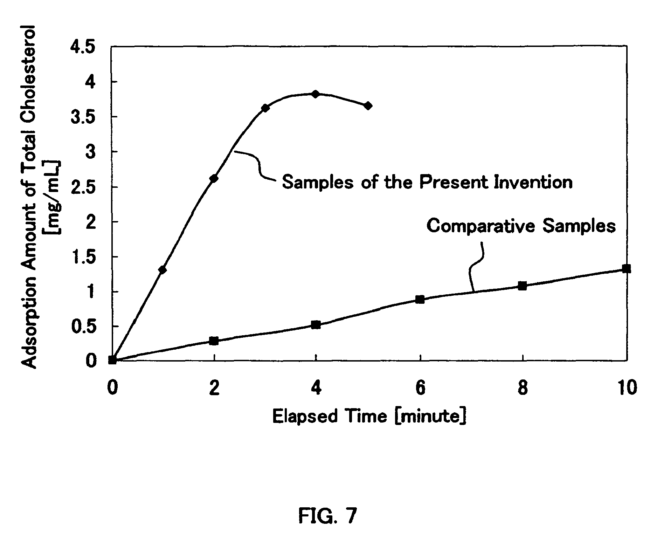

Also, referring to the test results shown in Fig. 5, from the test results shown in Fig. 7, the adsorption capacity of the total cholesterol was confirmed to have reached a saturated state in the samples , whereas the adsorption capacity of the total cholesterol was confirmed not to have reached a saturated state after five minutes, and further, after 25 minutes of the flowing-through time had passed in the comparative samples. In a confirmatory test in Fig. 7, fresh human plasma was flowed through another sample under the same conditions as applied to the samples S1 and S2 (an average flow velocity of 1 mL/minute), and 1 mL of eluted plasma was collected every minute, after which the adsorption capacity of the total cholesterol was obtained by the above-described Mathematical formula 1. Also, fresh human plasma was flowed through another comparative sample under the same conditions as applied to the comparative sample R1 (an average flow velocity of 1 mL/minute), and 2 mL of eluted plasma was collected every two minute, after which the adsorption capacity of the total cholesterol was obtained by the above-described Mathematical formula 1. As shown in Fig. 7, the sample has reached a saturated state after four minutes, whereas the comparative sample has not reached a saturated state even after 10 minutes.

From the test results shown in Figs. 5 and 7, a tendency was revealed such that the faster the average flow velocity was, the less the saturated adsorption amount was in the samples. Although a reason for the correlation between an average flow velocity and a saturated adsorption amount as noted above is unknown, it is suggested that, by increasing a flow velocity, the samples can exert its adsorbability of being able to reach the saturated adsorption amount in a short elution time with a sufficiently larger adsorption amount than that of the comparative samples. In other words, even if the saturated adsorption amount per adsorption column is reduced, body fluid purification can be conducted in a short time by increasing an average flow velocity, by which a burden imposed on a patient can be considerably reduced.

Also, while it was pointed out that a saturated adsorption amount of LDL is confined to approximately 6 mg/mL in an LDL adsorption column available at present in the PROBLEMS TO BE SOLVED BY THE INVENTION, neither the saturated adsorption amount of the samples nor the adsorption amount of the comparative examples has reached the saturated adsorption amount of approximately 6 mg/mL as converted to an adsorption amount of LDL according to the test results shown in Fig. 5. This is considered to be caused by that the average flow velocity in the above-described dynamic test method is high. It is readily predictable from the test results of Figs. 5 and 6 that the samples of the present invention would achieve the saturated adsorption amount sufficiently exceeding approximately 6 mg/mL if the average flow velocity is so low as to allow the adsorption amount of the comparative samples to reach the saturated adsorption amount.

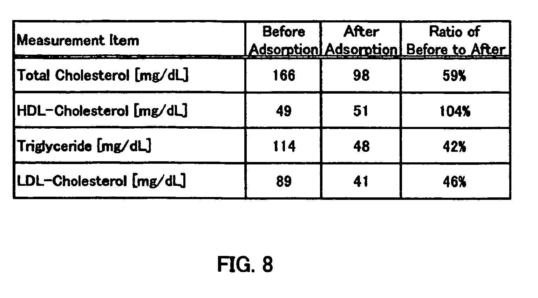

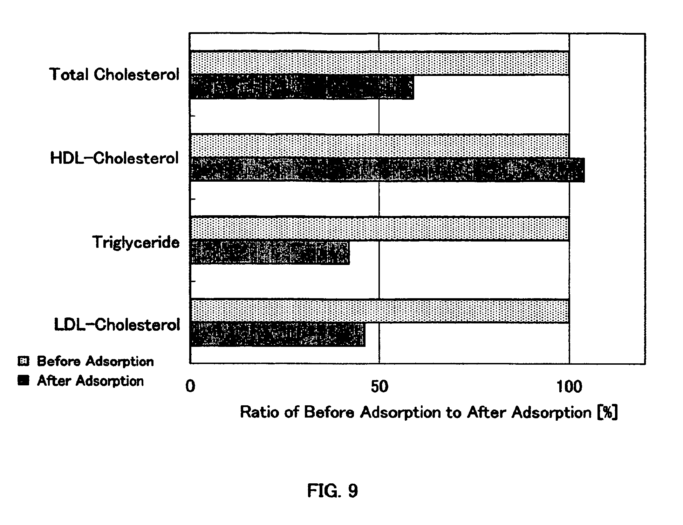

Then, the test results with regard to the adsorption selectivity of the samples are shown in the table in Fig. 8 and the graph in Fig. 9 as the concentrations before and after adsorption (mg/dL) and ratios thereof before and after adsorption (%) of lipids (the total cholesterol, HDL, triglyceride, and LDL) with respect to the sample number S2 (having a diameter of the through-pore of 2 $\mu$m and a diameter of the micropore of 130 nm). Based on Figs. 8 and 9, it is understood that the sample selectively adsorbs LDL containing apolipoprotein B, while it does not adsorb HDL containing apolipoprotein A. Furthermore, triglyceride binding to LDL is decreased in accordance with adsorption of LDL. As a result, based on the adsorbability of the total cholesterol as shown in the test results in Figs. 5 and 6, an adsorbability with respect to LDL can be evaluated.

Then, a dependency of adsorbability of the samples with respect to LDL on an average diameter of each of the through-pore and the micropore is described. For evaluation experiments of the dependency, a plurality of samples of the adsorbent obtained through the above-described steps 1 to 6 having different average diameters of each of the through-pore and the micropore are filled, 1 mL each, in small glass column containers. The column containers thus prepared are served as test columns. At this point, with regard to the average diameter of each of the through-pore and the micropore, samples were prepared to include ones being out of an appropriate range of the apparatus of the present invention.

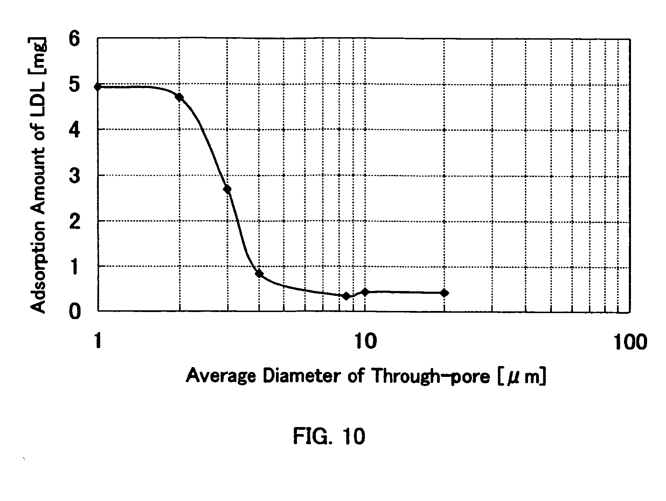

An evaluation test of a dependency of adsorbability of the samples with respect to LDL on an average diameter of the through-pore is conducted by flowing 5 mL of fresh human plasma at a flow velocity of 0.5 mL/minute through each test column once and analyzing the concentration of LDL in an eluted liquid. The adsorption amount of LDL (mg) is calculated by the above-described Mathematical formula 1 from the concentrations of LDL before and after adsorption for each test column having a varied average diameter of the through-pore within a range of 1 $\mu$m to 20 $\mu$m with respect to an average diameter of the micropore of 60 nm. Fig. 10 shows a property of the adsorption amount of LDL (saturated adsorption amount) in association with the diameter of the through-pore obtained by plotting the adsorption amount of LDL (mg) thus obtained against the average diameter of the through-pore ($\mu$m). As shown in Fig. 10, the adsorption amounts of LDL were 4.9 mg, 4.7 mg, 2.7 mg, 0.8 mg, 0.3 mg, 0.4 mg, and 0.4 mg with respect to the average diameters of the through-pore of 1 $\mu$m, 2 $\mu$m, 3 $\mu$m, 4 $\mu$m, 8.5 $\mu$m, 10 $\mu$m, and 20 $\mu$m, respectively.

From Fig. 10, it is understood that the average diameter of the through-pore is preferably in a range of 1 $\mu$m or more to less than 4 $\mu$m. A case in which the average diameter of the through-pore was less than 1 $\mu$m was not confirmed by an evaluation test because production of a sample itself was difficult, however, it is presumed that an efficiency of an LDL adsorption treatment will be reduced because a pressure loss of the adsorbent of the present invention becomes so high that flowing-through of the plasma at a desired flow velocity is interfered. In contrast, when the average diameter of the through-pore is 4 $\mu$m or more, the adsorption amount of LDL is reduced to less than 1 mg, and an effective surface area of the porous support is reduced; hence, it is presumed that an efficiency of LDL adsorption will be reduced.

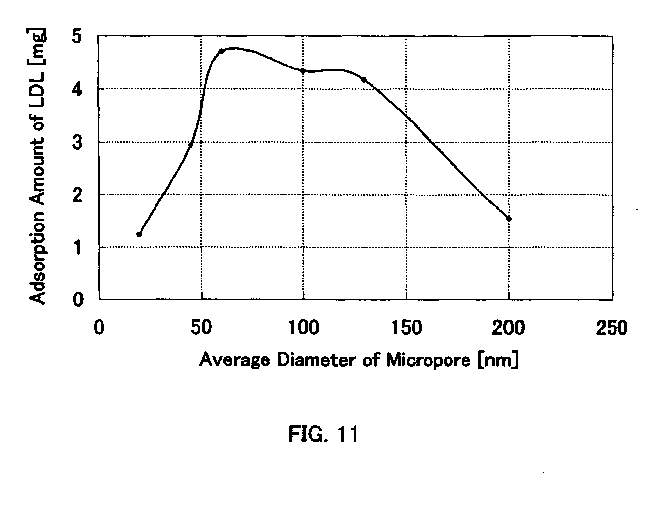

Then, an evaluation test of a dependency of adsorbability of the samples of the present invention with respect to LDL on an average diameter of the micropore is conducted by flowing 2 mL of fresh human plasma at a flow velocity of 0.5 mL/minute through each test column once and analyzing the concentration of LDL in an eluted liquid. The adsorption amount of LDL (mg) is calculated by the above-described Mathematical formula 1 from the concentrations of LDL before and after adsorption for each test column having a varied average diameter of the micropore within a range of 20 nm to 200 nm with respect to an average diameter of the through-pore of 2 $\mu$m. Fig. 11 shows a property of the adsorption amount of LDL (saturated adsorption amount) in association with the diameter of the micropore obtained by plotting the adsorption amount of LDL (mg) thus obtained against the average diameter of the micropore (nm). As shown in Fig. 11, the adsorption amounts of LDL were 1.2 mg, 2.9 mg, 4.7 mg, 4.3 mg, 4.2 mg, and 1.5 mg with respect to the average diameters of the micropore of 20 nm, 45 nm, 60 nm, 100 nm, 130 nm, and 200 nm, respectively.

From Fig. 11, when the average diameter of the micropore is within 20 nm to 200 nm, the adsorption amount of LDL is 1 mg or more, and further, when the average diameter of the micropore is within approximately 45 nm to 150 nm, more preferable results are obtained such that the adsorption amount of LDL is 3 mg or more. Accordingly, a lower limit value of the average diameter of the micropore is preferably equal to or larger than the diameter of LDL (26 to 27 nm) as a substance to be adsorbed, and further, more preferably approximately twice (approximately 50 nm) or more as large as the diameter of LDL. Also, an upper limit value of the average diameter of the micropore is preferably 200 nm or less, and further, more preferably 150 nm or less.

Second embodiment

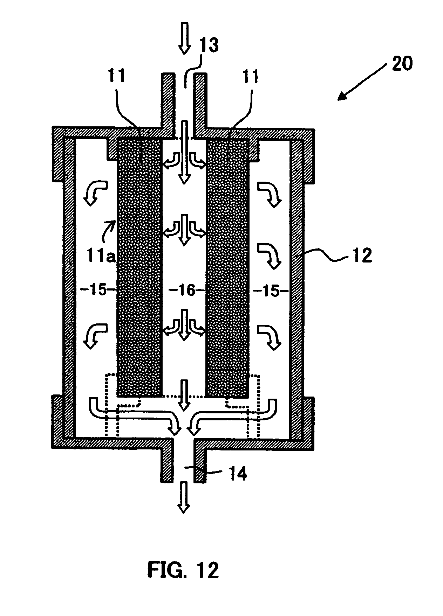

A second embodiment of the apparatus of the present invention is described. As shown in Fig. 12, an apparatus of the present invention 20 of a second embodiment is constituted in such a way that a cylindrical adsorbent 11 is contained in a cylindrical container 12. Openings 13 and 14 are each formed in each of end surfaces of the cylindrical container 12, and the opening 13 serves as an inlet for a body fluid to be treated (postulated to be the blood in the present embodiment), while the other opening 14 serves as an outlet for a treated body fluid.

As shown in Fig. 12, a cylindrical outer lateral surface 11a of the adsorbents 11 is spaced from an inner wall surface of the cylindrical container 12, and therebetween a channel 15 is formed for a body fluid (postulated to be the plasma here) which has passed through the adsorbent 11. Also, a cylindrical inner space of the adsorbent 11 serves as a channel 16 for a body fluid (blood) which does not pass through the adsorbent 11. The opening 13 is communicated with the channel 16, and the opening 14 is communicated with both the channel 15 and the channel 16.

While the outer shape of the adsorbent 11, which is the main component part of the apparatus of the present invention 20, is cylindrically-formed, it is provided as a porous support having an integrated structure having a double pore structure composed of two kinds of pores (the through-pore and the micropore) with different average diameters having a functional group such as dextran sulfate which has an affinity to LDL immobilized on the surface thereof, and this is the same as the adsorbent 1 of the first embodiment of the present invention. Also, each of the average diameters of the through-pore and the micropore of the porous support is the same as the case of the porous support 5 of the first embodiment. Accordingly, methods for producing the adsorbent and the porous support and adsorbabilities thereof are the same as in the first embodiment; therefore, a duplicate description is omitted.

In the apparatus of the present invention 20 shown in Fig. 12, the blood prior to separation of the plasma containing blood cell components having a large particle size such as an erythrocyte and a leukocyte other than the plasma from the blood cells is flowed as-is from the opening 13. Since the average diameter of the through-pore is adjusted to be in a range of 1 $\mu$m or more to less than 4 $\mu$m in the present embodiment, an erythrocyte, a leukocyte, and the like having a large particle size is discharged from the opening 14 without passing through the adsorbent 11. The plasma containing a substance to be adsorbed such as LDL obtained after an erythrocyte, a leukocyte, and the like having a large particle size have been separated in an inner space of the adsorbent 11 passes through the adsorbent 11 from the inner space to the outside, and the plasma after a substance to be adsorbed has been adsorbed in the inside of the adsorbent 11 is discharged from the opening 14 via the channel 15. In other words, the blood cell components which are an erythrocyte, a leukocyte, and the like having a large particle size separated in the inner space of the adsorbent 11 and the plasma after a substance to be adsorbed has been adsorbed are joined in the opening 14 and discharged to the outside of the apparatus 20 of the present invention. According to the apparatus of the present invention 20, a substance to be adsorbed contained in the plasma which is discharged without passing through the inside of the adsorbent 11 can be gradually adsorbed by the adsorbent 11 by refluxing the blood discharged from the opening 14 to the opening 13 side and circulating it.

Accordingly, in the apparatus of the present invention 20 of the second embodiment, despite that the average diameter of the through-pore of the porous support is adjusted to be in a range of 1 $\mu$m or more to less than 4 $\mu$m, removal of a substance to be adsorbed by adsorption is made possible without separating the blood cell components from the blood to be treated.

Another embodiment of the apparatus of the present invention is described hereinbelow.

Although a circular shape and a torus shape were postulated as a cross-sectional shape of the adsorbent 1 and the cylindrical container 2 in the above-described first embodiment of the present invention, the cross-sectional shape is not limited to a circular shape and a torus shape. Also, a torus shape was postulated as a cross-sectional shape of the adsorbent 11 and the cylindrical container 12 in the above-described second embodiment of the present invention, the cross-sectional shape is not limited to a torus shape.

Further, a case in which the opening 13 (inlet) is communicated with the channel 16 and the opening 14 (outlet) is communicated with both the channel 15 and the channel 16 is exemplified in Fig. 12 in the above-described second embodiment; however, it is also possible to employ a structure in which the opening 13 (inlet) is communicated with the channel 15, and the blood flowed from the opening 13 is supplied to the channel 15 outside the adsorbent 11, and the plasma containing a substance to be adsorbed such as LDL obtained after an erythrocyte, a leukocyte, and the like having a large particle size has been separated in the channel 15 is passed through the adsorbent 11 from the outside to the inner space thereof, and the plasma after a substance to be adsorbed has been adsorbed in the inside of the adsorbent 11 is discharged from the opening 14 via the channel 16.

INDUSTRIAL APPLICABILITY

The adsorption column for body fluid purification according to the present invention can be utilized for an adsorption column for an apheresis treatment, which aims to remove a predisposing factor such as LDL in the blood by adsorption.

**Claims**

1. An adsorption column for body fluid purification comprising
   a porous support (5) having a functional group (6) immobilized on a surface of the porous support (5), the functional group (6) specifically binding to a substance to be adsorbed including at least a low density lipoprotein, wherein the porous support (5) includes:

   a skeleton (7) made of a silica gel or silica glass having a three-dimensional network structure;

through-pores (8 of an average diameter in a range of 1 μm or more to less than 4 μm as measured by a mercury intrusion method, the through-pores (8) being formed in voids of the skeleton (7) and having a three-dimensional network form; and

micropores (9) of an average diameter of larger than a maximum length of the substance to be adsorbed in the body fluid purification as measured by a mercury intrusion method, the micropores (9) penetrating from a surface of the skeleton to an inner part thereof and being dispersedly formed, wherein

the average diameter of the micropores (9) as measured by a mercury intrusion method is between 27 nm and 200 nm .

2. The adsorption column for body fluid purification according to claim 1, wherein
the average diameter of the micropores (9) as measured by a mercury intrusion method is twice or more as large as the maximum length of the substance to be adsorbed.

3. The adsorption column for body fluid purification according to claim 1, wherein
the functional group (6) is dextran sulfate or a salt thereof, a polyacrylic acid or a salt thereof, or another chained polyhydric acid or a salt thereof having an affinity to specifically bind to a low density lipoprotein.

4. The adsorption column for body fluid purification according to claim 1, wherein
the porous support (5) is synthesized by a spinodal decomposition sol-gel method.

5. The adsorption column for body fluid purification according to any one of claims 1 to 4, wherein
the porous support (5) is formed into a columnar shape and contained in a cylindrical container (12) in such a way that a columnar lateral surface of the porous support (5) is closely contacted with an inner wall surface of the cylindrical container (12), the cylindrical container (12) having an opening (13, 14) at least in a part of each of both end surfaces, and

the opening in one end surface of the cylindrical container (12) is communicated with the opening in other end surface thereof via the through-pores of the porous support (5).

6. The adsorption column for body fluid purification according to any one of claims 1 to 4, wherein
the porous support (5) is formed into a cylindrical shape and contained in a cylindrical container (12) in such a way that at least a part of a cylindrical outer lateral surface of the porous support (5) is spaced from an inner wall surface of the cylindrical container (12), the cylindrical container having an opening at least in a part of each of both end surfaces,

the opening in one end surface of the cylindrical container (12) is communicated with both of an outer lateral part and an inner lateral part of the porous support (5), and

the opening in other end surface of the cylindrical container (12) is communicated with either one of the outer lateral part and the inner lateral part of the porous support (5).

**Patentansprüche**

1. Adsorptionssäule zur Körperflüssigkeitsreinigung, umfassend
eine poröse Stütze (5) mit einer Funktionsgruppe (6), die auf einer Oberfläche der porösen Stütze (5) festgesetzt ist, wobei die Funktionsgruppe (6) spezifisch an eine Substanz bindet, die adsorbiert werden soll, darunter zumindest ein Lipoprotein mit niedriger Dichte, wobei:

die poröse Stütze (5) Folgendes enthält:

ein Gerippe (7), das aus Kieselgel oder Kieselglas hergestellt ist, mit einer dreidimensionalen Netzwerkstruktur;
Durchgangsporen (8) mit einem durchschnittlichen Durchmesser von 1 μm oder mehr bis weniger als 4 μm bei Messung durch ein Quecksilberdruckverfahren, wobei die Durchgangsporen (8) in Hohlräumen des Gerippes (7) ausgebildet sind und eine dreidimensionale Netzwerkform aufweisen; und
Mikroporen (9) mit einem durchschnittlichen Durchmesser von mehr als der Höchstlänge der Substanz, die bei der Körperflüssigkeitsreinigung adsorbiert werden soll, bei Messung durch ein Quecksilberdruckvarfahren, wobei die Mikroporen (9) von einer Oberfläche des Gerippes zu einem Innenteil davon durchdringen und verstreut ausgebildet sind, wobei
der durchschnittliche Durchmesser der Mikroporen (9) bei Messung durch ein Quecksilberdruckverfahren zwischen 27 nm und 200 nm liegt.

2. Adsorptionssäule zur Körperflüssigkeitsreinigung nach Anspruch 1, wobei
der durchschnittliche Durchmesser der Mikroporen (9) bei Messung durch ein Quecksilberdruckverfahren zwei oder mehr Mal so groß wie die Höchstlänge der Substanz ist, die adsorbiert werden soll.

3. Adsorptionssäule zur Körperflüssigkeitsreinigung nach Anspruch 1, wobei
die Funktionsgruppe (6) Dextransulfat oder ein Salz davon, Polyacrylsäure oder ein Salz davon oder eine andere gekettete mehrwertige Säure oder ein Salz davon mit einer Affinität zum spezifischen Binden eines Lipoproteins mit niedriger Dichte ist.

4. Adsorptionssäule zur Körperflüssigkeitsreinigung nach Anspruch 1, wobei
die poröse Stütze (5) durch ein spinodales Sol-Gel-Entmischungs-Verfahren synthetisiert ist.

5. Adsorptionssäule zur Körperflüssigkeitsreinigung nach einem der Ansprüche 1 bis 4, wobei
die poröse Stütze (5) derart säulenförmig ausgebildet und in einem zylindrischen Behälter (12) enthalten ist, dass eine Säulenseitenfläche der porösen Stütze (5) eng mit einer Innenwandfläche des zylindrischen Behälters (12) in Kontakt steht, wobei der zylindrische Behälter (12) eine Öffnung (13, 14) zumindest in einem Teil von jeder von beiden Endflächen aufweist, und
die Öffnung in einer Endfläche des zylindrischen Behälters (12) über die Durchgangsporen der porösen Stütze (5) mit der Öffnung in der anderen Endfläche davon in Verbindung ist.

6. Adsorptionssäule zur Körperflüssigkeitsreinigung nach einem der Ansprüche 1 bis 4, wobei
die poröse Stütze (5) derart säulenförmig ausgebildet und in einem zylindrischen Behälter (12) enthalten ist, dass zumindest ein Teil einer zylindrischen Außenseitenfläche der porösen Stütze (5) von einer Innenwandfläche des zylindrischen Behälters (12) beabstandet ist, wobei der zylindrische Behälter eine Öffnung zumindest in einem Teil von jeder von beiden Endflächen aufweist,
die Öffnung in einer Endfläche des zylindrischen Behälters (12) mit sowohl einem Außenseitenteil als auch einem Innenseitenteil der porösen Stütze (5) in Verbindung ist, und
die Öffnung in der anderen Endfläche des zylindrischen Behälters (12) mit jedem des Außenseitenteils und des Innenseitenteils der porösen Stütze (5) in Verbindung ist.

**Revendications**

1. Colonne d'adsorption pour la purification de fluide corporel comprenant
un support poreux (5) comportant un groupe fonctionnel (6) immobilisé sur une surface du support poreux, le groupe fonctionnel (6) se liant spécifiquement à une substance devant être adsorbée incluant au moins une lipoprotéine basse densité, où
le support poreux (5) inclut :

un squelette (7) constitué d'un gel de silice ou d'un verre de silice ayant une structure en réseau tridimensionnelle ;
des pores traversants (8) d'un diamètre moyen dans la gamme de 1 $\mu$m ou plus à moins de 4 $\mu$m tel que mesuré par un procédé de pénétration de mercure, les pores traversants (8) étant formés dans des vides du squelette (7) et ayant une forme de réseau tridimensionnelle, et
des micropores (9) d'un diamètre moyen plus grand qu'une longueur maximale de la substance devant être adsorbée au cours de la purification de fluide corporel tel que mesuré par un procédé de pénétration de mercure, les micropores (9) pénétrant par une surface du squelette dans une partie interne de celui-ci et étant formés de façon dispersée, où
le diamètre moyen des micropores (9) tel que mesuré par un procédé de pénétration de mercure se situe entre 27 nm et 200 nm.

2. Colonne d'adsorption pour la purification de fluide corporel selon la revendication 1, dans laquelle
le diamètre moyen des micropores (9) tel que mesuré par un procédé de pénétration de mercure est deux fois supérieur ou plus à la longueur maximale de la substance devant être adsorbée.

3. Colonne d'adsorption pour la purification de fluide corporel selon la revendication 1, dans laquelle

le groupe fonctionnel (6) est le sulfate de dextrane ou un sel de celui-ci, un poly(acide acrylique) ou un sel de celui-ci, ou un autre acide polyhydrique à chaîne ou un sel de celui-ci ayant une affinité pour se lier spécifiquement à une lipoprotéine basse densité.

4.  Colonne d'adsorption pour la purification de fluide corporel selon la revendication 1, dans laquelle
    le support poreux (5) est synthétisé par un procédé sol-gel de décomposition spinodale.

5.  Colonne d'adsorption pour la purification de fluide corporel selon l'une quelconque des revendications 1 à 4, dans laquelle
    le support poreux (5) est de forme colomnaire et est contenu dans un contenant cylindrique (12) de sorte qu'une surface latérale colomnaire du support poreux (5) est en contact étroit avec une surface de paroi interne du contenant cylindrique (12), le contenant cylindrique (12) comportant une ouverture (13, 14) au moins dans une partie de chacune des deux surfaces d'extrémité, et
    l'ouverture dans une surface d'extrémité du contenant cylindrique (12) communique avec l'ouverture dans l'autre surface d'extrémité de celui-ci via les pores traversants du support poreux (5).

6.  Colonne d'adsorption pour la purification de fluide corporel selon l'une quelconque des revendications 1 à 4, dans laquelle
    le support poreux (5) est de forme cylindrique et est contenu dans un contenant cylindrique (12) de sorte qu'au moins une partie d'une surface latérale externe cylindrique du support poreux (5) est séparée d'une surface de paroi interne du contenant cylindrique (12), le contenant cylindrique (12) comportant une ouverture (13, 14) au moins dans une partie de chacune des deux surfaces d'extrémité, et
    l'ouverture dans une surface d'extrémité du contenant cylindrique (12) communique à la fois avec la partie latérale externe et la partie latérale interne du support poreux (5), et
    l'ouverture dans l'autre surface d'extrémité du contenant cylindrique (12) communique avec l'une ou l'autre des parties latérales interne ou externe du support poreux (5).

FIG. 1

FIG. 2

FIG. 3

FIG. 4

| Sample Number | S1 | S2 | S3 | S4 | R1 | R2 |
|---|---|---|---|---|---|---|
| Average Flow Velocity [mL/minute] | 1 | 1 | 0.5 | 0.3 | 1 | 0.2 |
| Elution Time [minute] | 5 | 5 | 10 | 17 | 5 | 25 |
| Amount of Eluted Liquid [mL] | 5.5 | 5.5 | 5.5 | 5.5 | 5.9 | 5.9 |
| Total Cholesterol Concentration before Flowing Through [mg/dL] | 179 | 166 | 181 | 240 | 205 | 208 |
| Total Cholesterol Concentration after Flowing Through [mg/dL] | 95 | 98 | 90 | 93 | 164 | 135 |
| Adsorption Capacity for Total Cholesterol per mL of Adsorbent [mg/mL] | 3.7 | 2.9 | 4.1 | 6.9 | 0.6 | 2.4 |

FIG. 5

FIG. 6

FIG. 7

| Measurement Item | Before Adsorption | After Adsorption | Ratio of Before to After |
|---|---|---|---|
| Total Cholesterol [mg/dL] | 166 | 98 | 59% |
| HDL-Cholesterol [mg/dL] | 49 | 51 | 104% |
| Triglyceride [mg/dL] | 114 | 48 | 42% |
| LDL-Cholesterol [mg/dL] | 89 | 41 | 46% |

FIG. 8

FIG. 9

FIG. 10

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3254756 A
- JP 3039736 A
- JP 7041374 A
- US 4637994 A

**Non-patent literature cited in the description**

- **Nobutaka TANI.** Development of Blood Adsorption Device. *Japanese Journal of Medical Instrumentation,* 1988, vol. 58 (6), 266-273